# EUROPEAN PATENT APPLICATION

(11) **EP 4 410 384 A1**
(43) Date of publication of application: **07.08.2024**
(21) Application number: 22875947.8
(22) Date of filing: 21.09.2022
(51) Int. Cl.: A61Q 19/00, A61K 8/31, A61K 8/34

(54) **COSMETIC OIL AND COSMETIC CONTAINING SAME**

(30) Priority: 30.09.2021 JP 2021160364
(71) Applicant: NOF Corporation, Shibuya-ku Tokyo 150-6019 (JP)
(72) Inventor: SATO Megumi, Kawasaki-shi Kanagawa 210-0865 (JP); SEKIGUCHI Koji, Kawasaki-shi Kanagawa 210-0865 (JP)
(74) Representative: Mewburn Ellis LLP
(86) International application number: PCT/JP2022/035056
(87) International publication number: WO 2023/054077

(57) **Abstract**

An object is to provide a cosmetic oil agent, capable of providing good affinity to the skin, emollient feel and powdery feel and of suppressing slimy feel when getting wet with water, and a cosmetic containing the same.

The cosmetic oil agent contains 30 to 65 mass% of a following component (A), 35 to 70 mass% of a component (B) and 0.1 to 20 mass% of a component (C). A ratio ((A)/(B)) of a mass of the component (A) with respect to a mass of the component (B) is 0.5 to 2. A ratio [((A)+(B))/(C)] of a total of the masses of the component (A) and the component (B) with respect to a mass of the component (C) is 10 to 200.
The component (A): a straight-chain saturated hydrocarbon having a carbon number of 8 to 13
The component (B): a straight-chain saturated hydrocarbon having a carbon number of 14 to 20
The component (C): a monovalent alcohol having a carbon number of 16 to 24

## Description

### TECHNICAL FIELD

The present invention is to provide a cosmetic oil capable of providing comfortable affinity to the skin, emollient feel and powdery feel and of suppressing slimy feel when getting wet with water, and a cosmetic containing the same.

### BACKGROUND OF THE INVENTION

Until now, volatile oils and silicone oils have been blended in may products, such as skin care cosmetics, emulsified cosmetics, make-up cosmetics, anti-sunburn cosmetics, cleansing cosmetics or hair care cosmetics, as feel-improving agents.

Generally, as usability for the feel-improving agent, it is listed to facilitate the affinity to the skin of a product, to suppress sticky feel of the product, and to further impart powdery feel (the feel of continuing sliding feel provided by a film formed on the skin after the application).

As volatile oil agents, such as cyclomethicone, light isoparaffin or isododecane, facilitate the fit on the skin of a product and impart light usability thanks to the volatility, various volatile oil agents have been developed. For example, according to patent document 1, it is disclosed hydrocarbon mixture of hydrocarbon having a carbon number of 11 and hydrocarbon having a carbon number of 13.

However, the volatile oil agents are problematic in that the emollient feel is poor due to the high volatility. Further, recently, many kinds of products have been commercialized in which a cosmetic is used under wet condition such as bath accompanied with increased diversity of cosmetics. When the cosmetic is used under the wet condition, it is necessary to suppress the slimy feel and to present slip on the viewpoint of safety of a consumer in the case that the cosmetic is applied and wet with water. Generally, as the slimy feel tends to be induced with a high blending ratio of an oil agent, it has been demanded to develop an oil agent capable of suppressing slimy feel when getting wet with water.

Thus, according to patent document 2, it is disclosed paraffin mixture with excellent volatility, which may be suitably used as a cosmetic or cleansing oil agent applied on skin or hair and which contains paraffins including isoparaffin having a carbon number of 12 to 16 and 2, 2, 4, 6, 6-pentamethyl heptane.

### [Prior technical documents]

### [Patent documents]

[Patent document 1] Japanese patent publication No. 2010-530387A
[Patent document 2] WO 2013/118533 A1

### SUMMARY OF THE INVENTION

### [Object to be solved by the invention]

However, according to the mixture of patent document 2, it is obtained the paraffin mixture with good affinity to the skin and excellent emollient feel. However, as the emollient feel is improved, the powdery feel may be insufficient. Further, there is a problem that the suppression of the slimy feel when getting wet with eater is not solved yet.

An object of the present invention is to provide a cosmetic oil agent, capable of providing good affinity to the skin, emollient feel and powdery feel and of suppressing slimy feel when getting wet with water, and to provide a cosmetic containing the same.

### [Solution for the object]

The present inventors have intensively researched the above items and found that the above problems can be solved by combining predetermined amounts of specific straight-chain saturated hydrocarbons and a specific monovalent alcohol.

That is, the present invention is as follows.
(1) A cosmetic oil agent comprising 30 to 65 mass% of a following component (A), 35 to 70 mass% of a component (B) and 0.1 to 20 mass% of a component (C),
   wherein a ratio ((A)/(B)) of a mass of the component (A) with respect to a mass of the component (B) is 0.5 to 2, and
   wherein a ratio [((A)+(B))/(C)] of a total of the masses of the component (A) and the component (B) with respect to a mass of the component (C) is 10 to 200.

   The component (A): a straight-chain saturated hydrocarbon having a carbon number of 8 to 13
   The component (B): a straight-chain saturated hydrocarbon having a carbon number of 14 to 20
   The component (C): a monovalent alcohol having a carbon number of 16 to 24
(2) A cosmetic containing 1 to 60 mass% of the cosmetic oil agent of (1).

### [Effects of the Invention]

According to the present invention, it is possible to obtain a cosmetic oil agent capable of providing good affinity to the skin, emollient feel and powdery feel, and of suppressing slimy feel when getting wet with water, and to obtain a cosmetic containing the same.

### EMBODIMENTS FOR CARRYING OUNT THE INVENTION

Embodiments of the present invention will be described below.

The present invention contains a component (A), component (B) and component (C), which will be described below in the order.

### [Component (A)]

As a straight-chain saturated hydrocarbon having a carbon number of 8 to 13 of the component (A) used in the present invention, although a kind of a raw material is not particularly limited as far as it can be conventionally blended in a cosmetic, a straight-chain saturated hydrocarbon having a carbon number of 11 to 13 is more preferred, and a straight-chain saturated hydrocarbon having a carbon number of 12 is particularly preferred.

As specific examples of the component (A), n-octane, n-nonane, n-decane, n-undecane, n-dodecane, n-tridecane and the like are listed. These components (A) can be used alone or in combination.

The content of the component (A) is made 30 to 65 mass%, provided that a total content of the cosmetic oil agent is made 100 mass%. In the case that the content of the component (A) exceeds 65 mass%, the affinity to the skin and emollient feel may possibly be deteriorated. The content is thus made 65 mass% or lower, is preferably 60 mass% or lower and is most preferably 55 mass% or lower. Further, in the case that the content of the component (A) is lower than 30 mass%, the powdery feel is insufficient and the slimy feel when getting wet with water may possible by provided. The content is thus made 30 mass% or higher, is preferably 35 mass% or higher, is more preferably 45 mass% or higher and is most preferably 50 mass% or higher.

Further, provided that 100 mass% is assigned to a total content of the cosmetic oil agent, on the viewpoint of stimulating property on skin and odor of the oil agent, the content of the straight-chain saturated hydrocarbon having a carbon number of 10 or lower may preferably be 10 mass% or lower and more preferably be 5 mass% or lower.

### [Component (B)]

As the straight-chain saturated hydrocarbon having a carbon number of 14 to 20 of the component (B) used in the present invention, although a kind of a raw material is not particularly limited as far as it can be conventionally blended in a cosmetic, a straight-chain saturated hydrocarbon having a carbon number of 14 to 18 is preferred, a straight-chain saturated hydrocarbon having a carbon number of 14 to 16 is more preferred, and a straight-chain saturated hydrocarbon having a carbon number of 14 is most preferred.

As specific examples of the component (B), n-tetradecane, n-pentadecane, n-hexadecane, n-heptadecane, n-octadecane, n-nonadecane, n-eicosane and the like may be listed. These components (B) may be used alone or in combination.

Provided that 100 mass% is assigned to a total content of the cosmetic oil agent, the content of the component (B) is made 35 to 70 mass%. In the case that the content of the component (B) is lower than 35 mass%, the emollient feel is insufficient. The content is thus made 35 mass% or higher, and more preferably be 40 mass% or higher. Further, in the case that the content of the component (B) is higher than 70 mass%, the affinity to the skin and powdery feel might be deteriorated and the slimy feel may possibly be provided when getting wet with water. The content is thus made 70 mass% or lower, is preferably 65 mass% or lower and is more preferably 50 mass% or lower.

### [Component (C)]

As the monovalent alcohol having a carbon number of 16 to 24 of the component (C) used in the present invention, although a kind of a raw material is not particularly limited as far as it can be conventionally blended in a cosmetic, on the viewpoint of the provision of the powdery feel and absence of the slimy feel when getting wet with water, the alcohol having a freezing point of 20°C °C or lower is preferred, the alcohol having a freezing point of 10°C or lower is more preferred and the alcohol having a freezing point of 0°C or lower is most preferred.

Further, the carbon number of the monovalent alcohol of the component (C) may preferably be 16 to 22, more preferably be 18 to 22 and most preferably be 18 to 20. Further, the monovalent alcohol of the component (C) may be a straight-chain alcohol or branched-chain alcohol.

Specific examples of the component (C) may be cetanol, cetearyl alcohol, stearyl alcohol, behenyl alcohol, hexyl decanol, isostearyl alcohol, 2-octyl-1-dodecanol, 2-decyl-1-tetradecanol and the like may be listed, isostearyl alcohol and 2-octyl-1-dodecanol are preferred, and 2-octyl-1-dodecanol is more preferred. These components (C) may be used alone or in combination.

In the case that 100 mass% is assigned to a total content of the cosmetic oil agent, the content of the component (C) is made 0.1 to 20 mass%. In the case that the content of the component (C) is lower than 0.1 mass%, the affinity to the skin and absence of slimy feel when getting wet with water are insufficient. The content is thus made 0.1 mass% or higher, is preferably 0.25 mass% or higher, is more preferably 0.5 mass% or higher and is most preferably 0.8 mass% or higher. Further, in the case that the content of the component (C) exceeds 20 mass%, the affinity to the skin, emollient feel, powdery feel and absence of slimy feel when getting wet with water may possibly be deteriorated. The content is thus made 20 mass% or lower, is preferably 15 mass% or lower, is more preferably 10 mass% or lower and is most preferably 5 mass% or lower.

According to the present invention, the mass ratio ((A)/(B)) of a mass of the component (A) with respect to a mass of the component (B) is made 0.5 to 2, on the viewpoint of affinity to the skin, emollient feel and powdery feel. In the case that the mass ratio ((A)/(B)) is lower than 0.5, the affinity to the skin and powdery feel are deteriorated. The mass ratio is thus made 0.5 or higher, is preferably 0.8 or higher, is more preferably 1 or higher and is most preferably 1.05 or higher. In the case that the mass ratio ((A)/(B)) exceeds 2, the powdery feel is deteriorated and the slimy feel may possibly be provided when getting wet with water. The mass ratio is thus made 2 or lower and is more preferably 1.6 or lower.

The mass ratio [((A)+(B))/(C)] of a total of the mass of the component (A) and mass of the component (B) with respect to the mass of the component (C) is made 10 to 200, is preferably 20 to 180, is more preferably 30 to 150 and is most preferably 50 to 120, on the viewpoint of the affinity to the skin, powdery feel and absence of slimy feel when getting wet with water.

### (Materials for cosmetic)

The cosmetic oil agent of the present invention can be applied in a cosmetic by blending into the cosmetic as a raw material. Although the applications of the inventive cosmetic oil agent are not limited, it may be suitably applied in, for example, a face cream, body cream, sun screen agent, moisturizing cream, hand cream, body cream, foot cream, massage cream, emulsion, foundation, oil beauty serum, massage oil, hair oil, cleansing oil and the like. Particularly, in the case it is blended in the face cream, body cream, sun screen agent, moisturizing cream, hand cream, body cream, foot cream, massage cream, emulsion, foundation and the like, the affinity to the skin, emollient feel, powdery feel and absence of slimy feel when getting wet with water can be further intensified.

The cosmetic oil agent of the present invention is applied as a feeling improving agent in a cosmetic, and the blending amount may preferably be 1 to 60 mass%, more preferably be 1 to 50 mass% and most preferably be 1 to 40 mass%. In the case that the blending amount of the cosmetic oil agent is lower than 1 mass%, sufficient effects might not be provided, and in the case that the amount is above 60 mass%, the stability of a formulation might possibly be deteriorated.

Further, into the cosmetic of the present invention, other ingredients generally applied in cosmetics, such as oils and fats, higher fatty acids, higher alcohols, silicones, esters, surfactants, moisturizers, thickening agents, anti-oxidants, stabilizers, preservatives, pearling agents, pH adjusting agents, ultraviolet ray absorbers, hydrotropes, pigments, coloring agents, fungicides, antiinflammatory agents, astringents, refreshing agents, fragrances, plant essences and the like, may be optionally added. Further, as a solvent, optionally, water, ethanol, 1,3-butylene glycol, dipropylene glycol, propylene glycol, glycerin and the like may be applied.

### EXAMPLES

The present invention will be described in detail below, referring to inventive examples and comparative examples.

As cosmetic oil agents, compositions shown in tables 1 and 2 were prepared. Further, as emulsified cosmetics with blended cosmetic oil agents, compositions shown in tables 3 and 4 were prepared. Each of them was evaluated according to the following methods and the results were shown in tables 1 to 4. Common added ingredients shown in tables 3 and 4 were shown in table 5.

### (Items and standards for evaluation)

### (1. affinity to the skin)

20 females (age of 22 to 40) were selected as panelists, and the affinity to the skin was evaluated based on the following standard in the case that the cosmetic oil agent or emulsified cosmetic (about 1 g) containing the same was applied. In the case that the result of "Ω" or "O" was obtained, it was judged that the cosmetic oil agent and emulsified cosmetic had good affinity to the skin.
2 points: When it is felt that the affinity to the skin is very good upon application on the skin
1 point: When it is felt that the affinity to the skin is relatively good upon application on the skin
0 point: When it is felt that the affinity to the skin is somehow bad upon application on the skin

### (5-grade evaluation based on a total of evaluation points)

Ω: The total of the evaluation points is 35 points or higher.
O: The total of the evaluation points is 30 points or higher and 34 points or lower.
Δ: The total of the evaluation points is 20 points or higher and 29 points or lower.
×: The total of the evaluation points is 19 points or lower.

### (2. Emollient feel)

20 females (age of 22 to 40) were selected as panelists. The emollient feel was evaluated based on the following standard when the cosmetic oil agent or emulsified cosmetic (about 1 g) containing the same was applied. In the case that the result of "O" or "Ω" was obtained, the cosmetic oil agent or emulsified cosmetic was judged as having excellent emollient feel.
2 points: It is felt that the emollient feel is very good upon application on the skin.
1 point: When it is felt that the emollient feel is relatively good upon application on the skin
0 point: when it is felt that the emollient feel is somehow bad upon application on the skin

### (5-grade evaluation based on a total of the evaluation points)

Ω: The total of the evaluation points is 35 points or higher.
O: The total of the evaluation points is 30 points or higher and 34 points or lower.
Δ: The total of the evaluation points is 20 points or higher and lower than 29 points.
×: The total of the evaluation points is 19 points or lower.

### (3. Powdery feel)

20 females (age of 22 to 40) were selected as panelists. The powdery feel was evaluated based on the following standard when the cosmetic oil agent or oil cosmetic (about 1 g) containing the same was applied. In the case that the result of "O" or "Ω" was obtained, the cosmetic oil agent and emulsified cosmetic were judged as having excellent powdery feel.
2 points: When it is felt that the powdery feel is very good upon application on the skin
1 point: When it is felt that the powdery feel is relatively good upon application on the skin
0 point: When it is felt that the powdery feel is somehow bad upon application on the skin

### (5-grade evaluation based on a total of the evaluation points)

Ω: The total of the evaluation points is 35 points or higher.
O: The total of the evaluation points is 30 points or higher and 34 points or lower.
Δ: The total of the evaluation points is 20 points or higher and 29 points or lower.
×: The total of the evaluation points is 19 points or lower.

### (3. Absence of slimy feel when getting wet with water)

20 females (age of 22 to 40) were selected as panelists. The cosmetic oil agent or emulsified cosmetic (about 3g) containing the same was applied on the bottom of foot after bathing, and the slimy feel was evaluated based on the following standard. In the case that the result of "O" or "Ω" was obtained, it was judged that the cosmetic oil agent or emulsified cosmetic was free from the slimy feel when getting wet with water.
2 points: In the case that slimy feel is not felt when getting wet with water
1 point: In the case that slimy feel is somehow felt when getting wet with water
0 point: In the case that slimy feel is felt when getting wet with water

### (5-grade evaluation based on a total of evaluation points)

Ω: The total of the evaluation points is 35 points or higher.
O: The total of the evaluation points is 30 points or higher and 34 points or lower.
Δ: The total of the evaluation points is 20 points or higher and 29 points or lower.
×: The total of the evaluation points is 19 points or lower.

**Table1**

| | | Inventive examples (mass%) | | | | | | |
|---|---|---|---|---|---|---|---|---|
| | | 1 | 2 | 3 | 4 | 5 | 6 | 7 |
| (A) | n-octane | 1 | 5 | 0.5 | | | | |
| | n-decane | | 5 | 0.5 | | | | |
| | n-undecane | 2.5 | | | | 13.5 | | |
| | n-dodecane | 55 | 45 | 35 | 51 | 31.5 | 50 | 50 |
| | n-tridecane | 2.5 | | | | | | |
| (A') | iso-dodecane | | | | | | | |
| (B) | n-tetradecane | 34.5 | 34.2 | 61 | 48 | 52.5 | 35 | 42 |
| | n-pentadecane | 2 | 10 | | | | | |
| | n-hexadecane | 2 | | | | | 9 | |
| (B') | iso-tetradecane | | | | | | | |
| (C) | isostearyl alcohol | | | 2 | | | | |
| | 2-octyl-1-dodecanol | 0.5 | 0.8 | 1 | 1 | 2.5 | 6 | |
| | 2-decyl-1-tetradecanol | | | | | | | 4 |
| | Stearyl alcohol | | | | | | | 4 |
| | Total | 100 | | | | | | |
| | Mass of (A) | 61 | 55 | 36 | 51 | 45 | 50 | 50 |
| | Mass of (B) | 38.5 | 44.2 | 61 | 48 | 52.5 | 44 | 42 |
| | ((A)+(B))/(C) | 199.0 | 124.0 | 32.3 | 99.0 | 39.0 | 15.7 | 11.5 |
| Evaluation items | Affinity to the skin | 0(31) | Ω(38) | O(33) | Ω(39) | O(32) | O(34) | O(33) |
| | Emollient feel | O(33) | O(34) | Ω(38) | Ω(39) | Ω(38) | Ω(39) | Ω(39) |
| | Powdery feel | O(32) | O(34) | 0(31) | Ω(39) | Ω(34) | O(31) | O(30) |
| | Absence of slimy feel upon wet with water | O(33) | Ω(37) | O(32) | Ω(39) | Ω(35) | Ω(36) | O(30) |

**Table 2**

| | | Comparative examples (mass%) | | | | | |
|---|---|---|---|---|---|---|---|
| | | 1 | 2 | 3 | 4 | 5 | 6 |
| (A) | n-octane | | | | | | |
| | n-decane | | | | | | |
| | n-undecane | | | | 30 | | |
| | n-dodecane | | 40 | | | 80 | 35 |
| | n-tridecane | | | 5 | 30 | | |
| (A') | iso-dodecane | | | 45 | | | |
| (B) | n-tetradecane | 75 | 60 | 40 | 20 | 20 | 40 |
| | n-pentadecane | 10 | | | | | |
| | n-hexadecane | 10 | | | | | |
| (B') | iso-tetradecane | | | | 15 | | |
| (C) | Isostearyl alcohol | | | 5 | | | 25 |
| | 2-octyl-1-dodecanol | 5 | | 5 | 5 | | |
| | 2-decyl-1-tetradecanol | | | | | | |
| | Stearyl alcohol | | | | | | |
| | Total | 100 | | | | | |
| | Mass of (A) | 0 | 40 | 5 | 60 | 80 | 35 |
| | Mass of (B) | 95 | 60 | 40 | 20 | 20 | 40 |
| | ((A)+(B))/(C) | 19.0 | - | 4.5 | 16.0 | - | 3.0 |
| Evaluation items | Affinity to the skin | Δ(20) | ×(18) | Δ(21) | O(30) | Δ(26) | Δ(23) |
| | Emollient feel | Ω(38) | Δ(25) | O(30) | Δ(29) | Δ(26) | Ω(39) |
| | Powdery feel | ×(15) | Δ(25) | Δ(21) | Δ(26) | Δ(23) | ×(10) |
| | Absence of slimy feel when wet with water | Δ(23) | ×(19) | Δ(27) | Δ(28) | ×(19) | Δ(20) |

**Table 3**

| | Inventive examples (mass%) | | | | |
|---|---|---|---|---|---|
| | 8 | 9 | 10 | 11 | 12 |
| Cosmetic oil agent of Inv. Ex. 2 | 30 | | | | |
| Cosmetic oil agent of Inv. Ex. 4 | | 50 | 30 | | |
| Cosmetic oil agent of Inv. Ex. 5 | | | | 20 | |
| Cosmetic oil agent of Inv. Ex. 7 | | | | | 5 |
| Common added components | 12 | | | | |
| Water | Residual part | | | | |
| Total | 100 | | | | |
| Affinity to the skin | Ω(38) | Ω(38) | Ω(39) | O(32) | O(32) |
| Emollient feel | O(34) | Ω(38) | Ω(39) | Ω(37) | Ω(38) |
| Powdery feel | O(34) | Ω(38) | Ω(39) | Ω(33) | O(30) |
| Absence of slimy feel when getting wet with water | Ω(36) | Ω(38) | Ω(39) | Ω(35) | O(30) |

**Table 4**

| | Comparative examples (mass%) | | | |
|---|---|---|---|---|
| | 7 | 8 | 9 | 10 |
| Cosmetic oil agent of Com. Ex. 2 | 20 | | | |
| Cosmetic oil agent of Com. Ex. 3 | | 20 | | |
| Cosmetic oil agent of Com. Ex. 4 | | | 20 | |
| Cosmetic oil agent of Com. Ex. 5 | | | | 20 |
| Common added components | 12 | | | |
| Water | Residual parts | | | |
| Total | 100 | | | |
| Affinity to the skin | ×(18) | Δ(21) | O(30) | Δ(26) |
| Emollient feel | Δ(25) | O(30) | Δ(29) | Δ(26) |
| Powdery feel | Δ(25) | Δ(21) | Δ(26) | Δ(23) |
| Absence of slimy feel when getting wet with water | ×(19) | Δ(27) | Δ (28) | ×(19) |

**Table 5**

| | mass% |
|---|---|
| Carbomer | 0.2 |
| Glycerin | 5 |
| Beeswax | 2.5 |
| PEG-75 Stearate | 1 |
| Polysorbate 80 | 1 |
| Glyceryl stearate | 2 |
| Phenoxytol | 0.3 |
| Total | 12 |

According to the inventive examples 1 to 12, the cosmetic oil agent containing the inventive components and cosmetic with the cosmetic oil agent blended therein were good in the provision of the affinity to the skin, emollient feel and powder feel and in absence of slimy feel when getting wet with water.

On the other hand, sufficient performance was not obtained in the comparative examples 1 to 10.

That is, according to the comparative example 1, as the component (A) is not blended, the affinity to the skin, powdery feel and absence of slimy feel when getting wet with water are insufficient.

According to the comparative examples 2, 5, 7 and 10, as the component (C) is not blended, the affinity to the skin, emollient feel, powdery feel and absence of slimy feel when getting wet with water are insufficient.

According to the comparative examples 3 and 8, as a part of the component (A) is replaced with other component (A'), the affinity to the skin, powdery feel and absence of slimy feel when getting wet with water are insufficient

According to the comparative examples 4 and 9, as the blending ratio of the component (B) is low, the affinity to the skin, emollient feel, powdery feel and absence of slimy feel when getting with water are insufficient.

According to the comparative example 6, as a high amount of the component (C) is blended, the affinity to the skin, powdery feel and absence of slimy feel when getting with water are insufficient.

### (Inventive example 13: Emulsion)

The respective components and additives are mixed in the composition described in the following table 6 to prepare emulsion. It is obtained the emulsified cosmetic (emulsion) capable of providing good affinity to the skin, emollient feel and powdery feel and of suppressing slimy feel when getting wet with water.

**Table 6**

| | Contents (mass%) |
|---|---|
| Cosmetic oil agent of inventive example 4 | 5% |
| Squalane | 2% |
| Dimethicone | 1% |
| Olive fruit oil | 2% |
| Ethyl hexyl palmitate | 2% |
| Glycerin | 5% |
| Butylene Glycol | 2% |
| Polyglyceryl-5 Stearate | 3% |
| PEG/PPG/ polybutylene glycol-8/5/3 glycerin | 0.5% |
| Polyquaternium-51 | 0.1% |
| Sodium Hyaluronate (1% aqueous solution) | 0.01% |
| Dipotassium Glycyrrhizate | 1% |
| Phenoxy ethanol | 0.3% |
| Ethyl hexyl glycerin | 0.1% |
| Carbomer | 0.3% |
| Potassium Hydroxide | Appropriate amount |
| Water | Residual part |
| Total | 100% |

### (Inventive example 14: Cream)

The respective components and additives are mixed, according to the composition described in the following table 7, to prepare cream. It is obtained emulsified cosmetic (cream) capable of providing good affinity to the skin, emollient feel and powdery feel and of suppressing slimy feel when getting wet with water.

**Table 7**

| | Contents (mass%) |
|---|---|
| Cosmetic oil agent of inventive example 4 | 3% |
| Squalane | 2% |
| Olive fruit oil | 2% |
| Ethyl hexyl palmitate | 2% |
| Glycerin | 5% |
| Propylene glycol | 3% |
| Butylene Glycol | 2% |
| Behenyl alcohol | 2% |
| Beeswax | 1% |
| Polyglyceryl-5 Laurate | 3% |
| PEG/PPG/ Polybutylene glycol-8/5/3 glycerin | 0.5% |
| Polyquaternium-51 | 0.1% |
| Sodium Hyaluronate (1% aqueous solution) | 0.01% |
| Tocopherol | 0.05% |
| Niacinamide | 3% |
| Phenoxy ethanol | 0.2% |
| Ethyl hexyl glycerin | 0.1% |
| Citric acid | Appropriate amount |
| Sodium Citrate | Appropriate amount |
| Fragrance | 0.05% |
| Water | Residual parts |
| Total | 100% |

### (Inventive example 15: Sunscreen agent)

The respective components and additives are mixed, according to the composition described in the following table 8, to prepare a sunscreen agent. It is obtained the sunscreen agent capable of providing the good affinity to the skin, emollient feel and powdery feel and of suppressing slimy feel when getting wet with water.

**Table 8**

| | Contents(mass%) |
|---|---|
| Cosmetic oil agent of inventive example 4 | 3% |
| Squalane | 2% |
| Dimethicone | 1% |
| Olive fruit oil | 2% |
| Ethyl hexyl palmitate | 2% |
| Disteardimonium Hectorite | 2% |
| (Acrylates/alkyl acrylate(C10-30)) cross polymer | 0.1% |
| Hydrogenated polyisobutene | 5% |
| Titanium oxide | 6% |
| Zinc oxide | 5% |
| Octyl methoxycinnamate | 2.8% |
| Diethylamino hydroxybenzoyl hexyl benzoat | 2.2% |
| PEG/PPG/polybutylene glycol-8/5/3 glycerin | 0.5% |
| Polyquaternium-51 | 0.1% |
| Glycerin | 5% |
| 1,3-butylene glycol | 2% |
| Sodium Hyaluronate (1% aqueous solution) | 0.01% |
| Phenoxy ethanol | 0.2% |
| Ethyl hexyl glycerin | 0.1% |
| Arginine | Appropriate amount |
| Water | Residual part |
| Total | 100% |

### (Inventive example 16: Point made remover)

The respective components and additive are mixed, according to the composition described in the following table 9, to prepare point make remover. It is obtained the point make remover capable of providing the good affinity to the skin, emollient feel and powdery feel and of suppressing slimy feel when getting wet with water.

**Table 9**

| | Contents (mass%) |
|---|---|
| Cosmetic oil agent of inventive example 4 | 10% |
| Cetyl ethylhexanoate | 15% |
| Caprylic/Capric Triglyceride | 1.5% |
| Sodium Chloride | 1% |
| Dipropylene Glycol | 7% |
| Glycerin | 3% |
| Pentylene glycol | 1% |
| Olive fruit oil | 1% |
| 1,2-hexane diol | 0.3% |
| Butylene Glycol | 1% |
| Disodium EDTA | 0.25% |
| Tocopherol | 0.05% |
| Water | Residual part |
| Total | 100% |

### (Inventive example 17: Oil cleansing)

The respective components and additives are mixed, according to the following table 10, to prepare an oil cleansing. It is obtained the oil cleansing capable of providing the good affinity to the skin, emollient feel and powdery feel and of suppressing slimy feel when getting wet with water.

**Table 10**

| | Contents (mass%) |
|---|---|
| Cosmetic oil agent of inventive example 4 | 20% |
| Cetyl ethyl hexanoate | 20% |
| Squalane | 5% |
| Olive fruit oil | 10% |
| Sorbeth tetra isostearate | 15% |
| Polyglyceryl-10 Diisostearate | 6% |
| Sorbitan oleate | 4% |
| Glyceryl laurate | 3% |
| Polysorbate 20 | 7% |
| Ethyl oleate | 6% |
| Tocopherol | 0.05% |
| Fragrance | 0.3% |
| Water | Residual part |
| Total | 100% |

### (Inventive example 18: Hair oil)

The respective components and additives are mixed, according to the composition described in the following table 11, to prepare a hair oil. It is obtained the hair oil capable of providing the good affinity to the skin, emollient feel and powdery feel and of suppressing slimy feel when getting wet with water.

**Table 11**

| | contents (mass%) |
|---|---|
| Cosmetic oil agent of inventive example 4 | 15% |
| Dimethicone | 65% |
| Olive fruit oil | 10% |
| Amino propyl dimethicone | 5% |
| Tocopherol | 0.05% |
| Fragrance | 0.3% |
| Water | Residual part |
| total | 100% |

### (Inventive example 19: Treatment)

The respective components and additives are mixed, according to the composition described in the following table 12, to prepare a treatment. It is obtained the treatment capable of providing the good affinity to the skin, emollient feel and powdery feel and of suppressing slimy feel when getting wet with water.

**Table 12**

| | contents (mass%) |
|---|---|
| Cosmetic oil agent of inventive example 4 | 5% |
| Behenyl trimethylammonium chloride | 1.5% |
| Stearamidopropyl dimethylamine | 0.5% |
| Squalane | 1% |
| Dimethicone | 5% |
| Panthenol | 0.5% |
| Amino propyl dimethicone | 1% |
| Cetyl Alcohol | 5% |
| Glycerin | 5% |
| Citric acid | Appropriate amount |
| Sodium Citrate | Appropriate amount |
| Phenoxy ethanol | 5% |
| Tocopherol | 0.05% |
| Fragrance | 0.3% |
| Water | Residual part |
| Total | 100% |

### (Inventive example 20: Mascara)

The respective components and additives are mixed, according to the composition described in the following table 13, to prepare a mascara. It is obtained the mascara capable of providing the good affinity to the skin, emollient fee and powdery feel and of suppressing slimy feel when getting wet with water.

**Table 13**

| | Contents (mass%) |
|---|---|
| Cosmetic oil agent of inventive example 4 | 20% |
| Iron oxides | 15% |
| Microcrystalline wax | 10% |
| Candelilla wax | 1.5% |
| Alcohol | 1% |
| Talc | 1% |
| Trimethylsiloxysilicate | 10% |
| Disteardimonium Hectorite | 2% |
| Dextrin palmitate | 1% |
| Propylparaben | 0.05% |
| Tocopherol | 0.05% |
| Hydrogenated polyisobutene | Residual part |
| Total | 100% |

### (Inventive example 21: Liquid foundation)

The respective components and additives are mixed, according to the composition described in the following table 14, to prepare a liquid foundation. It is obtained the liquid foundation capable of providing the good affinity to the skin, emollient feel and powdery feel and of suppressing slimy feel when retting wet with water.

**Table 14**

| | contents (mass%) |
|---|---|
| Cosmetic oil agent of inventive example 4 | 15% |
| Dimethicone | 15% |
| Beeswax | 1% |
| Hyaluronic acid Na | 0.01% |
| Polyquaternium-51 | 0.01% |
| Butylene Glycol | 5% |
| Dipropylene Glycol | 5% |
| Propane diol | 3% |
| Aluminum Hydroxide | Appropriate amount |
| Stearic acid | Appropriate amount |
| Tocopherol | 0.05% |
| Phenoxy ethanol | 0.5% |
| PEG-9 polydimethylsiloxyethyl dimethicone | 1% |
| Talc | 4% |
| Titanium oxide | 8% |
| Iron oxide | 2% |
| Water | Residual part |
| Total | 100% |

### (Inventive example 22: Lipstick)

The respective components and additives are mixed, according to the composition described in the following table 15, to prepare a lipstick. It is obtained the lipstick capable of providing the good sin fit, emollient feel and powdery feel and of suppressing slimy feel when getting wet with water.

**Table 15**

| | contents (mass%) |
|---|---|
| Cosmetic oil agent of inventive example 4 | 10% |

| Hydrogenated polyisobutene | Residual part |
|---|---|
| Castor oil | 5% |
| Jojoba oil | 5% |
| Olive oil | 5% |
| Carnauba wax | 2% |
| Polyethylene wax | 9% |
| Silicone resin | 18% |
| Dimethylpolysiloxane (SH100) | 5% |

| Aluminum Hydroxide | Appropriate amount |
|---|---|
| Red 201 | 1% |
| Titanium oxide | 2% |
| Pearl pigment | 5% |
| Total | 100% |

## Claims

1. A cosmetic oil agent comprising 30 to 65 mass% of a following component (A), 35 to 70 mass% of a component (B) and 0.1 to 20 mass% of a component (C),
wherein a ratio ((A)/(B)) of a mass of the component (A) with respect to a mass of the component (B) is 0.5 to 2, and
wherein a ratio [((A)+(B))/(C)] of a total of the masses of the component (A) and the component (B) with respect to a mass of the component (C) is 10 to 200.
The component (A): a straight-chain saturated hydrocarbon having a carbon number of 8 to 13
The component (B): a straight-chain saturated hydrocarbon having a carbon number of 14 to 20
The component (C): a monovalent alcohol having a carbon number of 16 to 24

2. A cosmetic containing 1 to 60 mass% of the cosmetic oil agent of claim 1.
